# EUROPEAN PATENT SPECIFICATION

(11) **EP 3 170 533 B1**
(45) Date of publication and mention of the grant of the patent: **01.04.2026**
(21) Application number: 15195035.9
(22) Date of filing: 17.11.2015
(51) Int. Cl.: A61P 17/02, A61K 9/06, A61K 47/02, A61K 47/10, A61K 47/36, A61L 26/00, A61K 31/734

(54) **COMPOSITION AND KIT FOR TREATMENT OF WOUNDS**
VERBINDUNG UND KIT ZUR BEHANDLUNG VON WUNDEN
COMPOSITION ET KIT POUR LE TRAITMENT DE PLAIES

(43) Date of publication of application: 24.05.2017
(73) Proprietor: Welcare Industries S.p.A. Società Benefit, 20121 Milano MI (IT)
(72) Inventor: Lazzarotto, Fulvia, 05018 Orvieto (IT); Zaru, Marco, 05018 Orvieto (IT)
(74) Representative: Di Giovine, Paolo

(56) References cited:
- US-A- 4 505 935
- US-A- 5 346 703
- NALBANDIAN R M ET AL: "PLURONIC F-127 GEL PREPARATION AS AN ARTIFICIAL SKIN IN THE TREATMENT OF THIRD-DEGREE BURNS IN PIGS", JOURNAL OF BIOMEDICAL MATERIALS RESEARCH, WILEY, NEW YORK, NY, US, vol. 21, no. 9, September 1987 (1987-09-01), pages 1135 - 1148, XP002915634, ISSN: 0021-9304, DOI: 10.1002/JBM.820210907

## Description

### STATE OF PRIOR ART

The treatment of skin lesions and wounds represents one of the most ancient problems for man and since ancient times one has looked for remedies for facilitating the skin tissue repair with consequent restore of a healthy and elastic skin.

One particular type of lesions, for example, is the one represented by skin lesions which may involve even the underneath tissue and wherein there are no open wounds, such as irritations, erythema, first-degree burns, wound healing phases wherein the skin has healed up or lesions caused by pressure such as for example decubitus ulcers or lesions. For the treatment of skin lesions, and in particular in case of lesions caused by pressure, such as decubitus plagues or in the process for healing wounds, ulcers, burns, scars, involving the skin and the underneath tissue, several factors are are extremely important, thereamong:
- cleaning and creation of an environment unfavourable to the microbial growth;
- capability of protecting the lesion and maintaining the right humidity degree without performing any occlusion;
- capability of absorbing exudates and contemporary haemostatic action mediated by the release of calcium ions.

Furthermore, several of the treatments known in the state of art are mediated by components with bactericidal or bacteriostatic activity, these components have several disadvantages such as the occurrence of resistance or other side effects dangerous for the patient.

US 4 505 935 A discloses a method of producing a protective layer on damaged skin, wherein a sodium alginate ointment is first applied on the skin and in a second passage a calcium salt solution is applied on top of said ointment resulting in an insoluble calcium alginate film.

Then, the problem of providing new compositions to be used in the treatment of the skin lesions is much felt.

### SUMMARY OF THE INVENTION

The present invention is based upon the mechanism of *in situ* crosslinking the sodium alginate mediated by calcium ions and the exploitation thereof in vulnologic and dermatologic field. In more details, the invention relates to the use of a hydrophilic gel constituted by a solution characterized by the capability of cleaning and creating an environment unfavourable to the microbial proliferation of the lesion jellied with sodium alginate. Such gel, with soft consistency and easily spreadable, once applied on the lesion surface, following exactly the shape thereof, is subsequently wet with the crosslinking solution of calcium ions which determines the reticulation and the formation of a soft and hydrated structure, but with gummier consistency. The function of the present invention, apart from keeping the site clean and unsuitable to the microbial proliferation, allows absorbing the exudates, by maintaining the right humidity degree, at the same time allowing a good transpiration as it does not exert any occlusive effect, and performing a haemostatic action mediated by the release of calcium ions. The product according to the present invention is able to be used directly before the dry medication.

An object of the present invention is then to create a system capable of joining in a single product a series of functions nowadays available in different products by creating an undoubted advantage in terms of *compliance* for the patients apart from clearly time and costs. Upon creating this joining of however known functions, a particular aspect of novelty and innovativeness is given by the in-situ crosslinking thanks thereto it is possible obtaining a lesion cover exactly equal to the features of the surface thereof with no excesses or deficits, which guarantees to avoid pain and all those troublesome conditions associated to the use of laminae, plasters or whatever not exactly sized to the features of the lesion, meant both under the terms of contour and clearly depth. Such approach, then, together with the joining of *cleaning*/*debridement* functions, constitutes a real medication, functional even in protective terms.

One of the new main aspects of the present invention is exactly that of gathering up different known actions in a single therapeutic instrument characterized by the very high use simplicity. As far as the first item is concerned, the colloidal/hydrophilic gel solution capable of performing such action is a solution including *poloxamer* 188. The peculiarity of this solution which differentiates it from many products destined to this purpose is the effectiveness thereof even in absence of disinfectants, that is substances equipped with bacteriostatic or bactericidal activity. Such gel can be applied onto the wound with the purpose of acting for the correct cleaning thereof by leaving the lesion site clean and unsuitable to the microbial proliferation and, then, protected from possible infections. Once ended the cleaning phase and eliminated gauze cloths/buffers used for rubbing, the wound is additioned with a further product so that this covers perfectly the surface thereof without not protected angles or areas. The subsequent application on the fluid gel film of the crosslinking solution determines the in-situ polymerization, that is the transformation of the fluid gel which covered the wound into an extremely hydrated calcium alginate lamina, capable of performing at the same time the following actions:
- protecting the lesion area thanks to the half-gummy consistency thereof
- maintaining the right humidity degree without occluding the lesion
- acting as absorbing system for possible exudates
- performing haemostatic action and constituting together with bandage the definite medication.

The new in-situ crosslinking system belonging to the present invention creates the premises for a system capable of associating to a single intervention both the *cleaning* and *debridement* aspect and that of primary medication of the lesion, undoubtedly representing a new strategy capable of reducing time and costs of medication and surely increasing the patient *compliance.*

From a technical point of view the system is based upon the synergy of different components and a new approach of in-situ crosslinking which by exploiting the properties of the alginates create a soft film-forming semi-irreversible film constituted by Calcium Alginate which represents a valid component in the treatment of ulcers and skin lesions.

Even if one does not want to link the invention to any action mechanism, it is associated to two concomitant events, that is the presence of pre-crosslinked hydrocolloid of components active cleaning and deeply debriding the lesion by creating an environment unfavourable to the microbial proliferation and the capability of crosslinking by recovering the lesion with a soft semi-reversible polymeric film. The *in-situ* crosslinking, as said, determines the formation of a gel of Alginate Calcium which maintains a high content of water and therefore an extremely soft and flexible state. The water contained therein, by evaporating, creates a light dehydration of the film which gradually, in absence of *swelling* phenomena, becomes gummier and more compact. This last aspect is almost never reached as the calcium ions, main promoter and responsible for the reticulum can be hindered by the phosphate ions usually present in the lesion site and this determines, on one side, the release of calcium ions which encourage the haemostatic process, on the other side they activate the *swelling* phenomenon which rehydrates the polymeric film by capturing a portion of the exudates of the lesion area by keeping it dry but deeply hydrated and with the right humidity degree.

Such mechanism explains the reticulum semi-reversibility features which are activated almost only in presence of phosphate ions and then, for example, in presence of exudates, blood, serum, etc., wherein together with the polymeric swelling, the calcium release would be activated, in favour of a haemostatic effect.

The subject of the present invention is a combination of a hydrophilic gel comprising poloxamer and sodium alginate and an aqueous solution comprising calcium salts, in particular calcium chloride, for topic use in a method for treating a skin lesion, wherein said treatment method comprises a first passage for applying said hydrophilic gel on said skin lesion and a second passage for applying said aqueous solution on said hydrophilic gel.

It is also disclosed a composition obtainable by mixing an aqueous solution comprising calcium chloride with a hydrophilic gel comprising poloxamer and sodium alginate.

It is also disclosed a kit for the treatment of skin lesions comprising a hydrophilic gel including poloxamer and sodium alginate and an aqueous solution comprising calcium chloride.

### DETAILED DESCRIPTION OF THE FIGURES

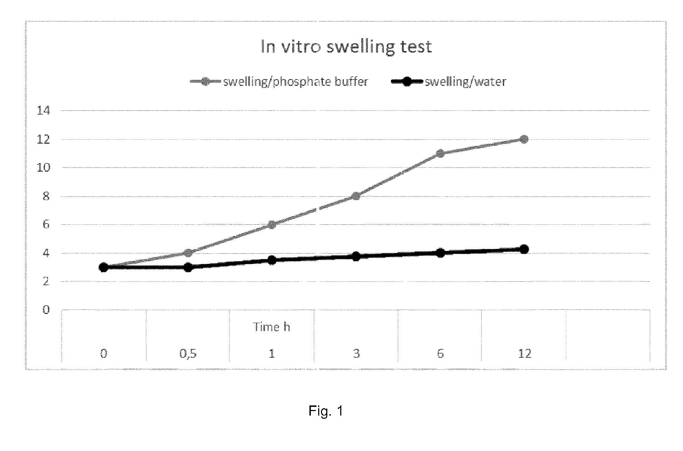

Figure 1: *Swelling* test. The figure shows the *swelling* effect versus time of a portion of crosslinked polymer, subsequently dehydrated for 1 hour at 4° degrees incubated in water (black line) or in phosphate buffer (grey line).

### DETAILED DESCRIPTION OF THE INVENTION

The present invention relates to a combination for topic use in a method for treating a skin lesion of a hydrophilic gel comprising poloxamer and sodium alginate and of an aqueous solution containing calcium salts such as for example, but not limited to, calcium chloride, calcium carbonate, calcium fluoride, calcium lactate or combinations thereof.

Said skin lesions are, for example, lesions from burns, abrasions, cuts, scratches, venous chronic ulcers, diabetic ulcers, arterial ulcers, decubitus ulcers, ulcers due to radiations, ulcers due to traumatic wounds.

The hydrophilic gel used in the present invention comprises poloxamer. The poloxamer is a copolymer soluble in water constituted by blocks of hydrophilic polyethylene oxide (PEO) and blocks of hydrophobic polypropylene oxide (PPO) connected therebetween. The nature of these block copolymers can be varied by checking the length of the PEO blocks and/or of the PPO block. Examples of poloxamer suitable to be used in the compositions of the present invention are poloxamer 188, poloxamer 407, poloxamer 127, poloxamer 237, poloxamer 235, poloxamer 335, poloxamer 405 and combinations thereof. Preferably poloxamer 188 will be used.

According to an embodiment the poloxamer of the hydrophilic gel is in a concentration comprised between 2 and 10% by weight, in particular between 4 and 6% preferably about 5%.

According to an embodiment the sodium alginate of the hydrophilic gel will be comprised between 1 and 5% by weight.

According to a particularly effective embodiment the hydrophilic gel will comprise poloxamer 188 at a concentration comprised between 4 and 6% and sodium alginate between 1 and 5% by weight.

According to an embodiment the hydrophilic gel could further comprise one or more compounds selected among Allantoin, Polysorbate 20, Linoleamidopropyl Pg-Dimonium Chloride Phosphate, Disodium EDTA, Sodium benzoate, Benzalkonium chloride, extract of leaves of *Aloe Barbadensis.* In particular allontoin.

The aqueous solution could comprise one or more preservatives compatible with the topic use known to the person skilled in the art, for example sodium benzoate, benzalkonium chloride, sodium dehydroacetate, phenoxyethanol, potassium sorbate, furthermore it could be a buffer solution, for example phosphate buffer for example with a pH between 5.5 and 6.

According to an embodiment the calcium chloride of the aqueous solution is comprised between 5 and 15% by weight.

According to a preferred embodiment the combination according to the present invention is without agents with bactericidal or bacteriostatic activity, in other words both the hydrophilic gel and the aqueous solution do not include active principles with bactericidal or bacteriostatic activity, for example antibiotics, sulfadiazine.

As described above the hydrophilic gel and the aqueous solution for use in a method for treating the skin lesions comprising a first passage of applying the hydrophilic gel on the skin lesion to be treated and a second passage of applying the aqueous solution on the hydrophilic gel and then on the lesion.

Compositions obtainable by preparing in a first passage the hydrophilic gel according to anyone of the above-described embodiments and then adding, for example by mixing or simply by pouring, the aqueous solution according to anyone of the above-described embodiments are also disclosed.

Kits for the treatment of the skin lesions comprising the hydrophilic gel according to anyone of the above-described embodiments and the aqueous solution according to anyone of the above-described embodiments are also disclosed.

According to the disclosure such kit could comprise one or more medical bottles including the herein described compositions and/or medical plasters, gauzes, bandages, tissues, absorbing buffers, at least partially soaked in the herein described compositions.

All products described herein could be made for example in form of medical device according to anyone of the classes described in the EC Directive 93/42 on the medical devices (which comprises even substances and not only "devices" in the mechanical sense of the word), or in any form according to the regulatory provisions of the country wherein such product will be implemented.

Also disclosed herein a method for the treatment of skin lesions comprising an application of the hydrophilic gel according to anyone of the above-described embodiments on the lesion to be treated and subsequently the aqueous solution according to anyone of the above-described embodiments.

All percentages of the substances shown in the present description are to be meant as percentage by weight, that is the percentages with respect to 100 g of the composition.

### EXAMPLES

Herebelow four formula examples are shown related to the hydrophilic gel by sake of simplicity designated as Uc-Med A and three formula examples related to the hydrophilic aqueous solution by sake of simplicity designated as Uc-Med B. Not limiting examples of composition according to the invention follow.

### Example 1 Formula related to 100 g of Uc - Med A1 prototype

| | |
|---|---|
| Water | 87.37 |
| *Aloe Barbadensis* leaf extract | 0.03 |
| Polysorbate 20 | 0.6 |
| Linoleamidopropyl Pg-Dimonium Chloride Phosphate | 1 |
| Allantoin | 0.4 |
| Disodium EDTA | 0.2 |
| Sodium benzoate | 0.15 |
| Benzalkonium chloride | 0.2 |
| Poloxamer 188 | 5.8 |
| Sodium alginate | 4.25 |

### Example 2 Formula related to 100 g of Uc - Med A2 prototype

| | |
|---|---|
| Water | 89.37 |
| *Aloe Barbadensis* leaf extract | 0.03 |
| Polysorbate 20 | 0.6 |
| Linoleamidopropyl Pg-Dimonium Chloride Phosphate | 1 |
| Allantoin | 0.4 |
| Disodium EDTA | 0.2 |
| Sodium benzoate | 0.15 |
| Benzalkonium chloride | 0.2 |
| Poloxamer 188 | 5.8 |
| Sodium alginate | 2.25 |

### Example 3 Formula related to 100 g of Uc-Med A3 prototype

| | |
|---|---|
| Water | 88.37 |
| *Aloe Barbadensis* leaf extract | 0.03 |
| Polysorbate 20 | 0.6 |
| Linoleamidopropyl Pg-Dimonium Chloride Phosphate | 1 |
| Allantoin | 0.4 |
| Disodium EDTA | 0.2 |
| Sodium benzoate | 0.15 |
| Benzalkonium chloride | 0.2 |
| Poloxamer 188 | 5.8 |
| Sodium alginate | 3.25 |

### Example 4 Formula related to 100 g of Uc- Med A 4 prototype

| | |
|---|---|
| Water | 90.37 |
| *Aloe Barbadensis* leaf extract | 0.03 |
| Polysorbate 20 | 0.6 |
| Linoleamidopropyl Pg-Dimonium Chloride Phosphate | 1 |
| Allantoin | 0.4 |
| Disodium EDTA | 0.2 |
| Sodium benzoate | 0.15 |
| Benzalkonium chloride | 0.2 |
| Poloxamer 188 | 5.8 |
| Sodium alginate | 1.25 |

### Example 5 Formula related to 100 g of Uc-Med B 1 prototype

| | |
|---|---|
| Water | 94.7% |
| Calcium chloride | 5 |
| sodium benzoate | 0.3 |

### Formula related to 100 g of Uc-Med B 2 prototype

| | |
|---|---|
| Water | 89.7% |
| Calcium chloride | 10 |
| sodium benzoate | 0.3 |

### Formula related to 100 g of Uc-Med B 3 prototype

| | |
|---|---|
| Water | 84.7% |
| Calcium chloride | 15 |
| sodium benzoate | 0.3 |

### TEST 1. EVALUATION OF THE SWELLING EFFECT OF THE INVENTION COMPOSITION

In order to evaluate the swelling features of the system originated by the *in-situ* crosslinking, a simple *in vitro* was made, wherein two samples constituted by 1 gr of Uc-Med A2 treated respectively with 100 microliters of Uc-Med B2 were first of all dehydrated and then incubated in two different solutions, that is in water and in phosphate buffer pH 7. As shown by figure 1 from the measurement of the sizes (thickness) of the polymer portion it is underlined that only in presence of phosphate ions it is possible detecting with a certain speed a real *swelling* effect. As said, this is due to the capability of the (bivalent) phosphate ions to hinder calcium ions and then to perform a de-crosslinking effect clearly connected to the ionic concentration itself. Such piece of data confirms what previously said, that is that in in presence of biological exudates and blood, the in-situ crosslinked system is capable of swelling by absorbing such fluids and at the same time releasing calcium in favour of a haemostasis strengthening. Analogous experiments were performed with the different formulas but the one which resulted more effective is the UC-Med A 2 formula prototype, in particular the Uc-Med A2 and Uc-Med B2 formula association.

The present invention has been sofar described with reference to some preferred embodiments. It is to be meant that other embodiments belonging to the same inventive core may exist, as defined by the protective scope of the herebelow reported claims.

## Claims

1. A combination of a hydrophilic gel comprising poloxamer and sodium alginate and an aqueous solution comprising one or more calcium salts for topic use in a method for treating a skin lesion, wherein said treatment method comprises a first passage for applying said hydrophilic gel on said skin lesion and a second passage for applying said aqueous solution on said hydrophilic gel.

2. The combination for use according to claim 1 wherein said poloxamer is selected among poloxamer 188, poloxamer 407, poloxamer 127, poloxamer 237, poloxamer 235, poloxamer 335, poloxamer 405 or combinations thereof.

3. The combination for use according to claim 1 or 2 wherein said calcium salt is selected among calcium chloride, calcium carbonate, calcium fluoride, calcium lactate or combinations thereof.

4. The combination for use according to anyone of claims 1 to 3 wherein said hydrophilic gel further comprises one or more compounds selected among Allantoin, Polysorbate 20, Linoleamidopropyl Pg-Dimonium Chloride Phosphate, Disodium EDTA, Sodium benzoate, Benzalkonium chloride, *Aloe Barbadensis* leaf extract.

5. The combination for use according to anyone of claims 1 to 4 wherein said poloxamer is in a concentration comprised between 2 and 10 % by weight of said hydrophilic gel.

6. The combination for use according to anyone of claims 1 to 5 wherein said calcium salt is calcium chloride comprised between 5 and 15% by weight of said aqueous solution.

7. The combination for use according to anyone of claims 1 to 6 wherein said sodium alginate is comprised between 1 and 5% by weight of said hydrophilic gel.

8. The combination for use according to anyone of claims 1 to 7 wherein said poloxamer is poloxamer 188 at a concentration comprised between 4 and 6%, said calcium salt is calcium chloride comprised between 5 and 15% and said sodium alginate is comprised between 1 and 5%.

9. The combination for use according to anyone of claims 1 to 3 wherein said hydrophilic gel and said aqueous solution are without agents with bactericidal or bacteriostatic activity.

10. The combination for use according to anyone of claims 1 to 9 wherein said lesions are burns, abrasions, cuts, scratches, venous chronic ulcers, diabetic ulcers, arterial ulcers, decubitus ulcers, ulcers due to radiations, ulcers due to traumatic wounds.

## Patentansprüche

1. Kombination aus einem hydrophilen Gel, umfassend Poloxamer und Natriumalginat, und einer wässrigen Lösung, umfassend ein oder mehrere Calciumsalze, zur topischen Verwendung in einem Verfahren zum Behandeln einer Hautläsion, wobei das Behandlungsverfahren einen ersten Schritt zum Auftragen des hydrophilen Gels auf die Hautläsion und einen zweiten Schritt zum Auftragen der wässrigen Lösung auf das hydrophile Gel umfasst.

2. Kombination zur Verwendung nach Anspruch 1, wobei das Poloxamer ausgewählt ist aus Poloxamer 188, Poloxamer 407, Poloxamer 127, Poloxamer 237, Poloxamer 235, Poloxamer 335, Poloxamer 405 oder Kombinationen davon.

3. Kombination zur Verwendung nach Anspruch 1 oder 2, wobei das Calciumsalz ausgewählt ist unter Calciumchlorid, Calciumcarbonat, Calciumfluorid, Calciumlactat oder Kombinationen davon.

4. Kombination zur Verwendung nach einem der Ansprüche 1 bis 3, wobei das hydrophile Gel ferner eine oder mehrere Verbindungen umfasst, die ausgewählt sind unter Allantoin, Polysorbat 20, Linoleamidopropyl Pg-Dimoniumchloridphosphat, Dinatrium EDTA, Natriumbenzoat, Benzalkoniumchlorid, *Aloe* Barbadensis-Blattextrakt.

5. Kombination zur Verwendung nach einem der Ansprüche 1 bis 4, wobei das Poloxamer in einer Konzentration ist, die zwischen 2 und 10 Gew.-% des hydrophilen Gels ausmacht.

6. Kombination zur Verwendung nach einem der Ansprüche 1 bis 5, wobei das Calciumsalz Calciumchlorid ist, das zwischen 5 und 15 Gew.-% der wässrigen Lösung ausmacht.

7. Kombination zur Verwendung nach einem der Ansprüche 1 bis 6, wobei das Natriumalginat zwischen 1 und 5 Gew.-% des hydrophilen Gels ausmacht.

8. Kombination zur Verwendung nach einem der Ansprüche 1 bis 7, wobei das Poloxamer Poloxamer 188 in einer Konzentration ist, die zwischen 4 und 6 % ausmacht, das Calciumsalz Calciumchlorid ist, das zwischen 5 und 15 % ausmacht und das Natriumalginat zwischen 1 und 5 % ausmacht.

9. Kombination zur Verwendung nach einem der Ansprüche 1 bis 3, wobei das hydrophile Gel und die wässrige Lösung ohne Mittel mit bakterizider oder bakteriostatischer Aktivität sind.

10. Kombination zur Verwendung nach einem der Ansprüche 1 bis 9, wobei die Läsionen Verbrennungen, Abschürfungen, Schnitte, Kratzer, venöse chronische Ulzera, diabetische Ulzera, arterielle Ulzera, Dekubitalulzera, durch Strahlung verursachte Ulzera, durch traumatische Wunden verursachte Ulzera sind.

## Revendications

1. Combinaison d'un gel hydrophile comprenant du poloxamère et de l'alginate de sodium et d'une solution aqueuse comprenant un ou plusieurs sels de calcium destinée à être utilisée par voie topique dans un procédé pour le traitement d'une lésion cutanée, dans laquelle ledit procédé de traitement comprend un premier passage pour l'application dudit gel hydrophile sur ladite lésion cutanée et un second passage pour l'application de ladite solution aqueuse sur ledit gel hydrophile.

2. Combinaison destinée à être utilisée selon la revendication 1, dans laquelle ledit poloxamère est choisi parmi poloxamère 188, poloxamère 407, poloxamère 127, poloxamère 237, poloxamère 235, poloxamère 335, poloxamère 405 ou combinaisons de ceux-ci.

3. Combinaison destinée à être utilisée selon la revendication 1 ou 2, dans laquelle ledit sel de calcium est choisi parmi chlorure de calcium, carbonate de calcium, fluorure de calcium, lactate de calcium ou combinaisons de ceux-ci.

4. Combinaison destinée à être utilisée selon l'une quelconque des revendications 1 à 3, dans laquelle ledit gel hydrophile comprend en outre un ou plusieurs composés choisis parmi allantoïne, polysorbate 20, phosphate de chlorure de linoléamidopropyl-PG-dimonium, EDTA disodique, benzoate de sodium, chlorure de benzalkonium, extrait de feuille *d'Aloe Barbadensis.*

5. Composition destinée à être utilisée selon l'une quelconque des revendications 1 à 4, dans laquelle ledit poloxamère est présent en une concentration comprise entre 2 et 10 % en poids dudit gel hydrophile.

6. Combinaison destinée à être utilisée selon l'une quelconque des revendications 1 à 5, dans laquelle ledit sel de calcium est du chlorure de calcium compris entre 5 et 15 % en poids de ladite solution aqueuse.

7. Combinaison destinée à être utilisée selon l'une quelconque des revendications 1 à 6, dans laquelle ledit alginate de sodium est compris entre 1 et 5 % en poids dudit gel hydrophile.

8. Combinaison destinée à être utilisée selon l'une quelconque des revendications 1 à 7, dans laquelle ledit poloxamère est le poloxamère 188 à une concentration comprise entre 4 et 6 %, ledit sel de calcium est le chlorure de calcium compris entre 5 et 15 % et ledit alginate de sodium est compris entre 1 et 5 %.

9. Combinaison destinée à être utilisée selon l'une quelconque des revendications 1 à 3, dans laquelle ledit gel hydrophile et ladite solution aqueuse sont dépourvus d'agents ayant une activité bactéricide ou bactériostatique.

10. Combinaison destinée à être utilisée selon l'une quelconque des revendications 1 à 9, dans laquelle lesdites lésions sont brûlures, écorchures, coupures, égratignures, ulcères veineux chroniques, ulcères diabétiques, ulcères artériels, ulcères de décubitus, ulcères dus à des radiations, ulcères dus à des plaies traumatiques.
